# EUROPEAN PATENT APPLICATION

(11) **EP 2 808 023 A1**
(43) Date of publication of application: **03.12.2014**
(21) Application number: 13169936.5
(22) Date of filing: 30.05.2013
(51) Int. Cl.: A61K 31/445, A61P 25/24

(54) **New drug for the treatment and/or prevention of depressive disorders**

(71) Applicant: Université Pierre et Marie Curie (Paris 6), 75005 Paris (FR); Centre National de la Recherche Scientifique (CNRS), 75794 Paris Cedex 16 (FR); Assistance Publique - Hôpitaux de Paris, 75184 Paris Cedex 04 (FR); UNIVERSITE PARIS DESCARTES, 75270 Paris Cedex 06 (FR)
(72) Inventor: Benoliel, Jean-Jacques, 75012 PARIS (FR); Becker, Christel, 75020 PARIS (FR); Bouvier, Elodie, 75018 PARIS (FR)
(74) Representative: Grosset-Fournier, Chantal Catherine

(57) **Abstract**

The present invention concerns a compound having the following formula (I): 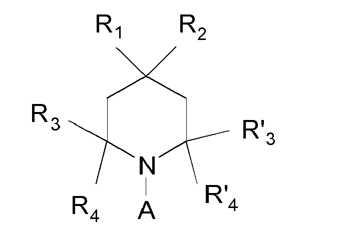 for its use as drug in the treatment and/or the prevention of depressions, with the proviso that said compound is not for use as drug in the treatment of women depressive state associated with premenstrual dysphoric disorder.

## Description

The present invention relates to a new drug for the treatment and/or prevention of depressive disorders.

Depression is a rather frequent mental disorder worldwide. It is believed to currently affect approximately 298 million people as of 2010, that means about 4.3% of the global population (Vos et al., Lancet. 2012 Dec 15;380(9859):2163-9).

Mood disorders can be classified into major depressive disorder, dysthymia, bipolar disorders, and some other humor troubles which can be induced by different factors, for example, by stressful events or drug abuse.

According to the National Institute of Mental Health, major depressive disorder is characterized by a combination of symptoms that interfere with a person's ability to work, sleep, study, eat, and enjoy once-pleasurable activities, while dysthymia is a chronic depression, with less severe but longer-lasting symptoms, which may persist for at least 2 years (Diagnostic and Statistical Manual of Mental Disorders-IV (DSM-IV)).

Bipolar depressive disorder, also known as manic-depressive illness, defines a brain disorder that causes unusual shifts in mood, energy, activity levels, and the ability to carry out day-to-day tasks. Individuals suffering bipolar disorder alternate episodes of a frenzied state, known as mania or hypomania, with episodes of depression. Bipolar disorders have several subtypes: bipolar I, bipolar II and cyclothymia (Diagnostic and Statistical Manual of Mental Disorders-IV (DSM-IV)).

It is to be noted that a depressive disorder, as a Major Depressive Disorder or a dysthymia, should not be confused with a depressive state which can appear in accompany with other physiological or psychological diseases, such as in the case of premenstrual dysphoric disorder in women.

In fact, a depressive state lasting less than a week and that does not need a special anti-depressive treatment, since it disappears with the relief of physiological or psychological pathology which is the cause. For example, depressive state in premenstrual dysphoric disorder does not need an anti-depressive treatment but only a hormonal treatment.

Till now, pathophysiological mechanisms of depressions remain poorly understand (Krishnan and Nestler, Nature, 2008, 455:894-902). Epidemiological, clinical, and basic research studies have revealed the existence of a complex interplay between genetic and environmental elements, with the physiological and psychological history of the subject playing a particularly important role (McEwen and Stellar, 1993, Arch Intern Med 153:2093-2101; Jacobson and Cryan, 2007, Behav Genet 37:171-213). Sustained or repetitive stress in adulthood may induce directly the pathology or may trigger maladaptive changes in some individuals, producing a vulnerable phenotype and may act as a trigger for mechanisms that leave predisposed individuals at increased risk of illness development (de Kloet et al., 2005). Thus, psychological stressful events (e.g., death of relatives, divorce, humiliation or defeat, deterioration of financial status) may sensitize neurobiological systems, leading to a state of vulnerability to the subsequent development of depression later in life.

It is known that oxidative stress can be generated after a stressful event and associated to a psychiatric disorders including depression (Hovatta et al., 2010, Neurosci. Res. 68:261-275). Oxidative stress pathways provide a tempting target for pharmacological intervention. However, fewer studies examined the efficacy of antioxidant treatments on depressive disorders. Treatment with N-acetylcysteine (NAC), a glutathione precursor that effectively replenishes brain glutathione, induced a robust decrement in depression scores in patients suffering of bipolar depression (Berk et al., Neuro Endocrinol Lett., 32 (2):133-40, 2011). More studies are necessary for establishing that target oxidative-stress-related mechanisms may be beneficial in treatment of depressive disorders.

Currently, the most popular treatment of depression disorder is the administration of antidepressants, which primarily work on brain neurotransmitters, especially monoamine (serotonin, norepinephrine, or dopamine).

However, more and more clinical data show that currently available antidepressants have only a poor effectiveness for treating the depression (Fava et al., The American Journal of Psychiatry, 2006, 163 (7): 1161-72; Trivedi et al., New England Journal of Medicine, 2006, 354 (12): 1243-52; Rush et al., New England Journal of Medicine, 2006, 354 (12): 1231-42). It is reported that between 30% and 50% of individuals treated with a given available antidepressant do not show a positive response (Ruhé et al., The Journal of Clinical Psychiatry, 2006, 67 (12): 1836-55).

Consequently, there is an urgent need to develop more efficient antidepressants. Moreover, it is particularly interesting to develop a drug to prevent occurrence of depression in vulnerable high risk population.

TEMPOL (4-hydroxy-2,2,6,6-tetramethylpiperidin-1-oxyl) is known as an antioxidant and has been used as drug in the treatment of fibrocystic disease of breast, rectal hemorrhoids, periodontitis and/or gingivitis, migraine headache, cyclic vomiting syndrome, zoster and/or post-herpetic neuralgia, and premenstrual dysphoric disorder for women (US2012/0115905). However, the experiment reported in US 2012/0115905 is only carried out in one female patient in a very particular condition, wherein said patient has a long history for fibrocystic disease of breast, which is so severe that surgeons had recommended mastectomy and said patient had also been previously diagnosed as suffering from severe trigeminal neuralgia. So what is described in US 2012/0115905 is only an affirmation, but cannot be considered as a positive human clinical trial, even not as a significant result.

Recently, BDNF (brain derived neurotrophic factor), has been reported as a relevant biomarker for diagnosing high-risk individual within a population at risk (Blugeot et al., 2011, J. Neurosci. 31(36):12889-12899). It has been also shown that intracerebroventricular administration of 7,8-DHF, which is a BDNF mimetic, can prevent neuroanatomical alterations, such as hippocampal CA3 dendritic retraction and increase of amygdala dendritic length, and the occurrence of depressive profile.

However, how BDNF implies in the occurrence of depressive disorders is still unknown.

The subject-matter of the present invention is to provide compounds for their use as drug in the treatment and/or the prevention of depressions.

Particularly, the present invention concerns a compound having the following formula (I): 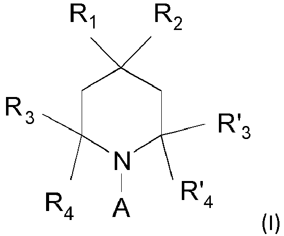 wherein:
- R₁ and R₂ represent independently from each other: H, ORₐ, wherein Rₐ represents H, a C₁-C₁₀-alkyl, aryl or heteroaryl group,
   or R₁ = R₂ and represent =O, =NR_{b} wherein R_{b} represents H, a C₁-C₁₀-alkyl, aryl or heteroaryl group, =CR_{c}R_{d}, wherein R_{c} and F_{d} represents independently from each other a C₁-C₁₀-alkyl, aryl or heteroaryl group,
- R₃ and R₄ represents independently form each other H, a C₁-C₁₀-alkyl, aryl or heteroaryl group,
- R'₃ and R'₄ represents independently form each other H, a C₁-C₁₀-alkyl, aryl or heteroaryl group,
- A represents OH or O⁻,
for its use as drug in the treatment and/or the prevention of depressions, with the proviso that said compound is not for use as drug in the treatment of women depressive state associated with premenstrual dysphoric disorder.

The present invention is based on the surprising experiment results obtained by the Inventors that TEMPOL can restore neuroanatomical alterations induced by psychological stressful events and prevents the development of depressive phenotype after stressful event in vulnerable group, that is to say that their serum or hippocampal BDNF level is constantly below than a normal control level.

The Inventors have established for the first time that BDNF is not only a relevant biomarker for diagnosing high-risk individual within a population at risk, but also can regulate oxidative stress by controlling Nrf2 activity, and that the alleviation of oxidative stress by an aforementioned compound of formula (I) can treat and/or prevent depressive disorders.

In fact, the experiments of the Inventors show that BDNF can activate Nrf2 translocation from cytosol to nucleus. Nuclear Nrf2 is implied in response to oxidative stress by binding to antioxidant response element (ARE), thus up-regulates a battery of antioxidant and detoxifying genes (Singh et al., 2010, Free Radical Research 44(11): 1267-1288).

The term "depression" refers to depressive disorders which can be major depressive disorder and dysthymia, or bipolar depressive disorders including BP I, BP II and cyclothymia.

In the frame of the present invention, the terms "depressions" and "depressive disorders" can be replaced one with another. The term "bipolar depression", "bipolar depressive disorder" and "bipolar disorder" can be replaced one with another.

The term "depressive state" refers to a depressive mood which lasts less than a week and can disappears without antidepressant treatment.

The term "premenstrual dysphoric disorder" refers to a female disorder characterized by serious premenstrual distress, and associated deterioration of social and emotional functioning. The symptoms of premenstrual dysphoric disorder, such as depression, anxiety, mood swings, may be linked to abnormal hormone level variation occurring during the hormonal cycle.

An abnormal hormone level in a woman suffering from premenstrual dysphoric disorder is often the origin of her depressive mood. However, depressive disorders in general, and in women can have other origins than abnormal hormone level.

It is to be understood that in the case of the present invention, the group of patients concerned excludes women suffering from premenstrual dysphoric disorder and manifesting depressive state.

But the group of patients does not exclude women suffering from depressive disorders not in relation to premenstrual dysphoric disorder.

The term "alkyl" refers to a linear or branched chain, saturated hydrocarbon having the indicated number of carbon atoms. A C₁-C₁₀ alkyl can include but is not limited to methyl, ethyl, propyl, isopropyl, n-butyl, isobutyl, sec-butyl, tert-butyl, n-pentyl, isopentyl, n-hexyl, n-heptyl, n-octyl, n-nonyl, n-decyl.

The term "aryl" refers to an unsubstituted aromatic system, such as a phenyl, or substituted aromatic system by at least one OH group, at least one (C₁-C₃) alkyl group.

The term "heteroaryl" refers to a monocyclic- or polycyclic aromatic ring comprising carbon atoms, hydrogen atoms, and one or more heteroatoms, preferably, 1 to 3 heteroatoms, independently selected from nitrogen, oxygen, and sulfur. In the context of the present invention, an heteroaryl group can include but is not limited to pyridinyl, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, phenyl, isoxazolyl, and oxazolyl. A heteroaryl group can be unsubstituted or substituted with one or two suitable substituents.

A particular embodiment of the invention concerns a compound of above defined formula (I) for its use as drug in the treatment and/or the prevention of depressions, with the proviso that said compound is not for use as drug in the treatment of women with major depressive disorder or dysthymia.

In a particular embodiment, the present invention concerns a compound of above-defined formula (I) for its use as drug in the treatment and/or the prevention of depression resistant to classical treatment.

The term "depression resistant to a classical treatment" means that three successive classical therapeutic treatments for depressive disorder in a patient are failures.

A therapeutic treatment is considered as failed if half of depressive symptoms are maintained more than 4 weeks of treatment.

A "classical treatment" means a treatment by commercially available antidepressants or by psychotherapy.

For example, said classical antidepressant can be selected from SSRI (selective serotonin reuptake inhibitor [as citalopram, escitalopram, fluoxetine, fluvoxamine...]), TCA (tricyclic antidepressant [as imipramine, desipramine, amitriptyline...]), MAOI (monoamine oxidase inhibitor [as Clorgyline, Moclobemide, Toloxatone..]), SNRI (serotonin-norepinephrine reuptake inhibitor [as venlafaxine, duloxetine]), NDRI (norepinephrine-dopamine reuptake inhibitor [as bupropion]), SARI (serotonin antagonist and reuptake inhibitor [as trazodone]), dopamine reuptake inhibitor [as amineptine], lithium.

A psychotherapy used in classical treatment of depression can be behavioral therapy and cognitive therapies.

In another particular embodiment, the present invention concerns a compound of above-defined formula (I) for its use as drug prescribed as initial treatment of a depression.

The term "initial treatment" means the first drug prescribed to a patient for treating a depressive disorder. This means that the compound of the present inventions is not intended to replace a classical treatment.

In a more particular embodiment, the present invention concerns a compound of above-defined formula (I) for its use as drug in the treatment of major depressive disorder or dysthymia in men or in women without premenstrual dysphoric disorder and bipolar depression in men and women and/or the prevention of depression in men and women.

In another more particular embodiment, the present invention concerns a compound of above-defined formula (I) for its use as drug in the treatment and/or the prevention of major depressive disorder or dysthymia in men and bipolar depressive disorders in men and women.

In a particular embodiment of the invention, the compound has formula (I), wherein:
- R₁ represents H and R₂ represents H or OH,
- R₃=R₄=R'₃=R'₄ and R₃, R₄, R'₃, and R'₄ represent a C₁-C₁₀ alkyl, in particular a methyl, an ethyl, a propyl, or an isopropyl,
- A represents OH or O'.

Another particular embodiment of the present invention is a compound for its use as drug in the treatment and/or the prevention of depressions, with the proviso that said compound is not used for the treatment of women depressive state associated with premenstrual dysphoric disorder, particularly not used for the treatment of women with major depressive disorder or dysthymia, said compound having the following formula (II): 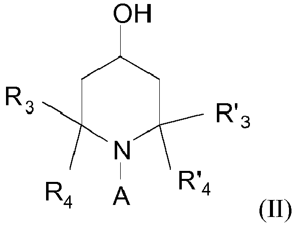 wherein A, R₃, R₄, R'₃, and R'₄ are as defined above.

In a preferable embodiment of the present invention, the compounds have the formula (II), wherein:
- R₃=R₄=R'₃=R'₄ and R₃, R₄, R'₃, and R'₄ represent a C₁-C₁₀ alkyl, in particular a methyl, an ethyl, a propyl, or an isopropyl,
- A represents OH or O'.

In a more particular embodiment, the present invention concerns a compound for its use as drug in the treatment and/or the prevention of depressions, with the proviso that said compound is not used for the treatment of women depressive state associated with premenstrual dysphoric disorder, particularly not used for the treatment of women with major depressive disorder or dysthymia, said compound having the following formula (III): 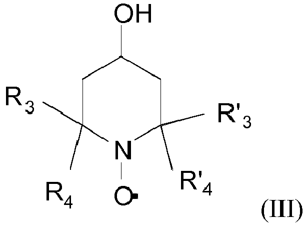 wherein R₃, R₄, R'₃, and R'₄ are as defined above.

In a preferable embodiment of the present invention, the compounds have the formula (III), wherein R₃=R₄= R'₃=R'₄ and R₃, R₄, R'₃, and R'₄ represent a C₁-C₁₀ alkyl, in particular a methyl, an ethyl, a propyl, or an isopropyl group.

In a more preferable embodiment of the present invention, the compounds have the formula (V):

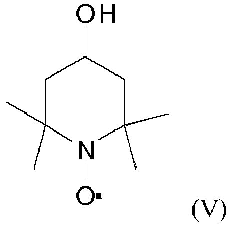

The compound of formula (V) is named also as TEMPOL.

Another more particular embodiment of the present invention is a compound for its use as drug in the treatment and/or the prevention of depressions, with the proviso that said compound is not used for the treatment of women depressive state associated with premenstrual dysphoric disorder, particularly not used for the treatment of women with major depressive disorder or dysthymia, said compound having the following formula (IV): 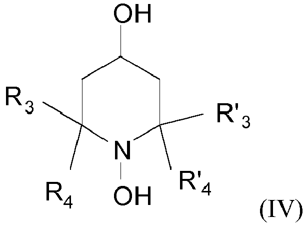 wherein:
R₃, R₄, R'₃, and R'₄ are as defined above.
In a preferable embodiment, the compounds of the present invention have the formula (IV), wherein R₃=R₄= R'₃=R'₄ and R₃, R₄, R'₃, and R'₄ represent a C₁-C₁₀ alkyl, in particular a methyl, an ethyl, a propyl, or an isopropyl.
TEMPO (2,2,6,6-tetramethyl-1-piperidinyl-1-oxyl), which is another compound of formula (I), can also be used as drug in the frame of the present invention for the treatment and/or the prevention of depressions.

Another embodiment of the present invention concerns an above defined compound, in particular the compound of formula (V), for its use as drug in the treatment and/or the prevention of depression, with the proviso that said compound is not for the treatment of women depressive state associated with premenstrual dysphoric disorder, particularly not used for the treatment of women with major depressive disorder or dysthymia, wherein said compound is liable to be chronically administered at a dose comprised from 0.1 mg/kg to 300 mg/kg, in particular from 10 mg/kg to 125 mg/kg.

A "chronic administration" defines a drug administration paradigm where the drug is administrated for more than one experimental session over the course of several days.

In another embodiment, the compound of the present invention is liable to be acutely administered at a dose comprised from 30 mg/kg to 275 mg/kg.

An "acute administration" defines a drug administration paradigm where a drug is given during one experimental session (once during the experiment, or multiple times) within a 24 hour period.

Said compound can be formulated in adequate form to be administered by oral or intravenous route.

Said pharmaceutical composition can comprise also a pharmaceutical acceptable vehicle.

A pharmaceutical composition, comprising an above mentioned compound, in particular the compound of formula (V) as active substance, can be formulated as tablets, pills or capsules for oral administration, or as a solution for intravenous administration.

When said pharmaceutical composition is for oral administration, it can comprise fillers, blenders, glidants, lubricants, disintegrants, flavous, colorants, sweeterners, and sorbants.

In a particular embodiment, the compound of the present invention, in particular the compound of formula (V), is used as drug in the treatment/and or prevention of depression afflicting a human patient who has a level of BDNF in a ratio of about 35% below that of a control patient, with the proviso that said compound is not used for the treatment of women depressive state associated with premenstrual dysphoric disorder, particularly not used for the treatment of women with major depressive disorder or dysthymia.

The decreasing level of BDNF after a psychological stressful event compared to a control patient is a biomarker of vulnerable high risk population.

The administration of a compound of the present invention, in particular the compound of formula (V), can efficiently alleviate oxidative stress and decrease the risk to develop a depressive disorder.

The term "control patient" refers to a patient who never has any type of depressive disorder history in his/her life.

BDNF level can be determined either in serum or in hippocampus, by the method described in Blugeot et al. (2011, J. Neurosci. 31(36):12889-12899).

In a more particular embodiment, the compound of the present invention, in particular the compound of formula (V), is used as drug in the treatment/and or prevention of depression afflicting a human patient who has a serum level of BDNF in a ratio of about 35% below that of a control patient also measured in serum, with the proviso that said compound is not used for the treatment of women depressive state associated with premenstrual dysphoric disorder, particularly not used for the treatment of major depressive disorder or dysthymia in women.

In another more particular embodiment, the compound of the present invention, in particular the compound of formula (V), is used as drug in the treatment/and or prevention of depression afflicting a human patient who has a level of the brain-derived neurotrophic factor (BDNF) measured in hippocampus in a ratio of about 35% below that of a control patient also measured in hippocampus, with the proviso that said compound is not used for the treatment of women depressive state associated with premenstrual dysphoric disorder, particularly not used for the treatment of major depressive disorder or dysthymia in women.

Another embodiment of the present invention concerns an aforementioned compound, in particular the compound of formula (V), for its use as drug in the treatment and/or prevention of depressive disorders afflicting a human patient who further has an oxidative stress which is determined by classical markers, such as physiological level of superoxide dismutase (SOD) and/or of lipoperoxidation (as tBARS [Thiobarbituric acid reactive substances], oxysterols..) and/or of gluthatione reductase (GSR) and/or of the ration GSH/GSSG (reduced glutathione/oxidized glutathione) and/or of protein oxidation and/or of gluthatione peroxidase (GPx) and/or catalase and/or Reactive Oxygen Species (ONOO⁻, *OH, NO, H₂O₂, O₂⁻) in said patient.

"Oxidative stress" corresponds to an impairment of the balance between oxidant production and the antioxidant capacity leading to increased levels of reactive oxygen species (ROS) and oxidative damages as lipid peroxidation.

The physiological level of above mentioned molecules in the frame of the present invention can be a molecular level measured in serum, plasma, red blood cells, cerebrospinal fluid (CSF) or brain tissue.

The level of SOD, tBARS, oxysterols, GSR, GPx, GSH/GSSG, protein oxidation, or ROS can be determined by any conventional methods, in particular the methods described below in Example. One skilled in the art understands that these molecules level in serum, plasma, red blood cells, cerebrospinal fluid (CSF) or brain tissue could be different and that it is preferable to measure the level of SOD, tBARS, oxysterols, GSR, GPx, GSH/GSSG, protein oxidation, or ROS in patient to be treated and that of normal control in the same type of biological sample.

A patient is considered as having an oxidative stress when the level of at least one molecule of SOD, tBARS, oxysterols, GSR, GPx, GSH/GSSG, protein oxidation, or ROS is different from the level in a normal control.

Preferably, the normal control of a given molecule is defined by the laboratory that performed the assay.

For example, a patient is considered as having an oxidative stress when his/her SOD level in red blood cells is higher than 950-1100 U/g of hemoglobin, and/or when his/her GSR level in red blood cells is higher than 8.4-8.87 U/g of hemoglobin, and/or when his/her GPx level in red blood cells is higher than 5.5-19 U/g of hemoglobin, and/or when his/her tBARS level in red blood cells is higher than 95-105 mmol/g of hemoglobin.

Another embodiment of the present invention concerns an aforementioned compound, in particular the compound of formula (V), for its use as drug in the treatment and/or prevention of depressions, in combination with an compound with antidepressant-like properties or an antidepressant drug, in particular selected from the group consisting of: 7,8 DHF (compound with antidepressant-like properties), SSRI (selective serotonin reuptake inhibitor, such as citalopram, escitalopram, fluoxetine, fluvoxamine, etc.), TCA (tricyclic antidepressant, such as imipramine, desipramine, amitriptyline, etc.), MAOI (monoamine oxidase inhibitor, such as Clorgyline, Moclobemide, Toloxatone, etc.), SNRI (serotonin-norepinephrine reuptake inhibitor, such as venlafaxine, duloxetine), NDRI (norepinephrine-dopamine reuptake inhibitor, such as bupropion]), SARI (serotonin antagonist and reuptake inhibitor, such as trazodone]), dopamine reuptake inhibitor (such as amineptine), lithium.

The present invention concerns also an aforementioned compound, in particular the compound of formula (V), for its use as drug in the treatment and/or prevention of depressions, said compound being liable to be administered during the implementation of a therapy selected from the group consisting of: electroshock therapy, electroconvulsive therapy (ECT), transcranial magnetic stimulation (TMS), repetitive transcranial magnetic stimulation (rTMS), behaviour therapy.

In another particular embodiment, the present invention concerns a compound of above-defined formula (I) for its use as drug in the prevention of depression in vulnerable population.

The term "vulnerable population" refers to a population, after stressful events, displaying persistent decreased serum BDNF concentrations. In animals, the serum BDNF levels decrease is associated with reduced hippocampal volume and neurogenesis, CA3 dendritic retraction and decrease in spine density, as well as amygdala neuron hypertrophy.

In a more particular embodiment, the present invention concerns compound of formula (V) for its use as drug in the prevention of depressive disorders in men and women.

The other subject-matter of the present invention is a pharmaceutical composition comprising at least an aforementioned compound, in particular the compound of formula (V), and at least another antidepressant drug, in combination with a pharmaceutically acceptable vehicle.

In particular, the antidepressant is an antidepressant, selected from the group consisting of: 7,8 DHF (compound with antidepressant-like properties), SSRI (selective serotonin reuptake inhibitor [as citalopram, escitalopram, fluoxetine, fluvoxamine...]), TCA (tricyclic antidepressant [as imipramine, desipramine, amitriptyline...]), MAOI (monoamine oxidase inhibitor [as Clorgyline, Moclobemide, Toloxatone..]), SNRI (serotonin-norepinephrine reuptake inhibitor [as venlafaxine, duloxetine]), NDRI (norepinephrine-dopamine reuptake inhibitor [as bupropion]), SARI (serotonin antagonist and reuptake inhibitor [as trazodone]), dopamine reuptake inhibitor [as amineptine], lithium.

In a particular embodiment of the above defined pharmaceutical composition, the weight proportion of the compound of the present invention is comprised from 50% to 85% and the weight proportion of said antidepressant drug is comprised from 50% to 15%.

The present invention concerns also a product of combination comprising at least an aforementioned compound, and at least another antidepressant drug, in particular selected from the group consisting of: 7,8 DHF (compound with antidepressant-like properties), SSRI (selective serotonin reuptake inhibitor [as citalopram, escitalopram, fluoxetine, fluvoxamine...]), TCA (tricyclic antidepressant [as imipramine, desipramine, amitriptyline...]), MAOI (monoamine oxidase inhibitor [as Clorgyline, Moclobemide, Toloxatone..]), SNRI (serotonin-norepinephrine reuptake inhibitor [as venlafaxine, duloxetine]), NDRI (norepinephrine-dopamine reuptake inhibitor [as bupropion]), SARI (serotonin antagonist and reuptake inhibitor [as trazodone]), dopamine reuptake inhibitor [as amineptine], lithium as a combination for the simultaneous, separate use or successive administration for the treatment and/or the prevention of depressive disorders.

More particularly, said product of combination is used as a combination for the simultaneous, separate use or successive administration for the treatment and/or the prevention of depressions, with the proviso that said product of combination is not used for women depressive state associated with premenstrual dysphoric disorder.

In a particular embodiment, the present invention concerns a product comprising a compound of formula (V) and at least another above mentioned antidepressant drug, as a combination for the simultaneous, separate use or successive administration for the treatment and/or the prevention of depressive disorders.

Particularly, said product of combination is used as a combination for the simultaneous, separate use or successive administration for the treatment and/or the prevention of depressive disorders, with the proviso that said product of combination is not used for women depressive state associated with premenstrual dysphoric disorder.

More particularly, said product of combination is used as a combination for the simultaneous, separate use or successive administration for the treatment and/or the prevention of depressive disorders, with the proviso that said product of combination is not used for women with major depressive disorder or dysthymia.

Said product can be used in the treatment and/or the prevention of a depression resistant to classical treatment or as drug prescribed as initial treatment of a depression, in particular major depressive disorder or dysthymia in men and women and bipolar depression in men and women.

More particularly, said product can be used in the treatment and/or the prevention of a depression, in particular major depressive disorder or dysthymia in men or in women without premenstrual dysphoric disorder and bipolar depression in men and women.

The present invention is illustrated, but is not limited to, the following figures and examples.

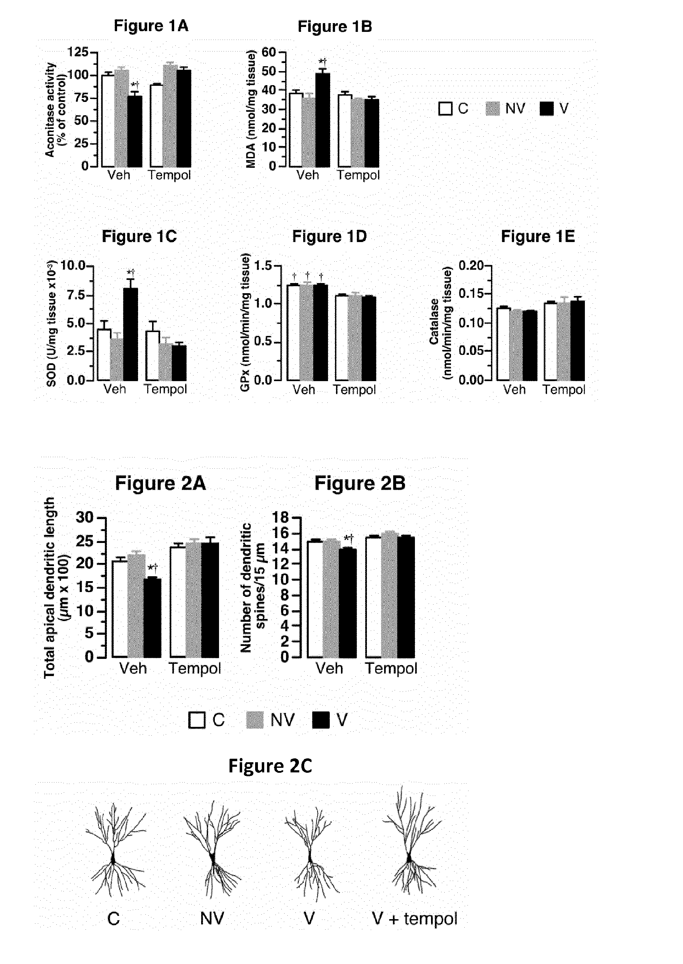

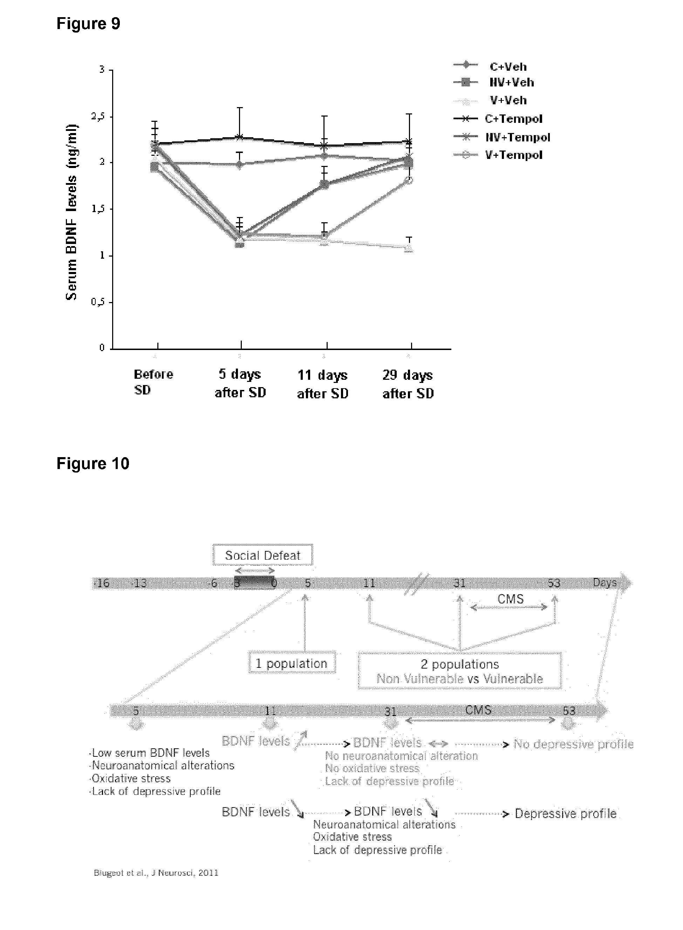

Figures 1A, 1B, 1C, 1D and 1E display the effect of TEMPOL on oxidative stress state estimated by the measurement of aconitase (Figure 1A, an enzyme whose activity reduces with oxidation state, of a lipoperoxidation (Figure 1B, MDA is a marker of oxidative stress state measured with the help of the kit from Cayman Chemical, Ann Arbor, MI, USA), of a SuperOxide Dismutase (Figure 1C, an enzyme implied in the conversion from superoxide ions to hydrogen peroxide, an increasing of enzyme activity means the increasing of hydrogen peroxide level), of gluthatione peroxidase (GPx, Figure ID) and of catalase (Figure 1E). TEMPOL is respectively administrated by systemic route during 15 days at a dose of 288 µmol/kg/day in three animal groups: control animals (group C), non-vulnerable group (NV, stressed animals identified as resilient) or vulnerable group (that is to say aware of an anterior stress, group V). The animals treated by TEMPOL are compared to those only treated by vehicle. The results of statistical analyses are presented in Table 1, wherein * P < 0.05 vs C animals; † P < 0.05 vs the corresponding tempol-treated group. The experiments concerned in figures 1A, 1B, 1C, 1D and 1E are carried out on 31 days after social defeat (see Figure 10).
Figures 2A, 2B and 2C show the effect of TEMPOL on hippocampus neuron's morphology appreciated by total apical dendrite length of CA3 neurons (Figure 2A, 2C) and dendritic spine number (Figure 2B). A morphological alteration of hippocampal neuron is carried out before the triggering of a depressive disorder and is considered as a residual trace which sensitizes the development of this disorder. C: control animal group treated by vehicle; NV: non-vulnerable group treated by vehicle; V: vulnerable group treated by vehicle; V+TEMPOL: vulnerable group treated by TEMPOL. The results of statistical analyses are presented in Table 1, wherein * P < 0.05 vs C animals; † P < 0.05 vs the corresponding tempol-treated group. The experiments concerned in figures 2A, 2B and 2C are carried out on 31 days after social defeat (see Figure 10).

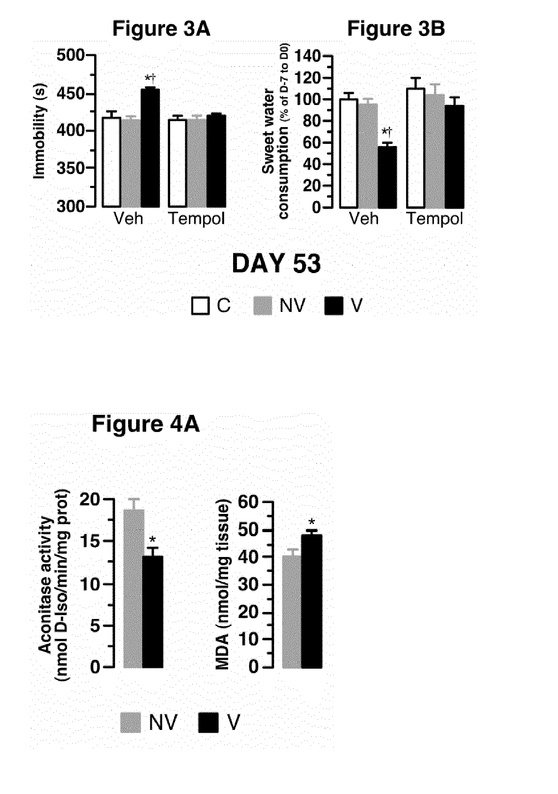

Figures 3A and 3B show the effect of TEMPOL on depressive phenotype. Depressive phenotype is evaluated by resignation behavior measured by forced swim test (Porsolt test) and sweet water consumption test (which can be assimilated as loss of pleasure, reflecting "anhedonia"). The results of statistical analyses are presented in Table 1, wherein * P < 0.05 vs C animals; † P < 0.05 vs the corresponding TEMPOL-treated group. The experiments concerned in figures 3A and 3B are carried out on 53 days after social defeat (see Figure 10).

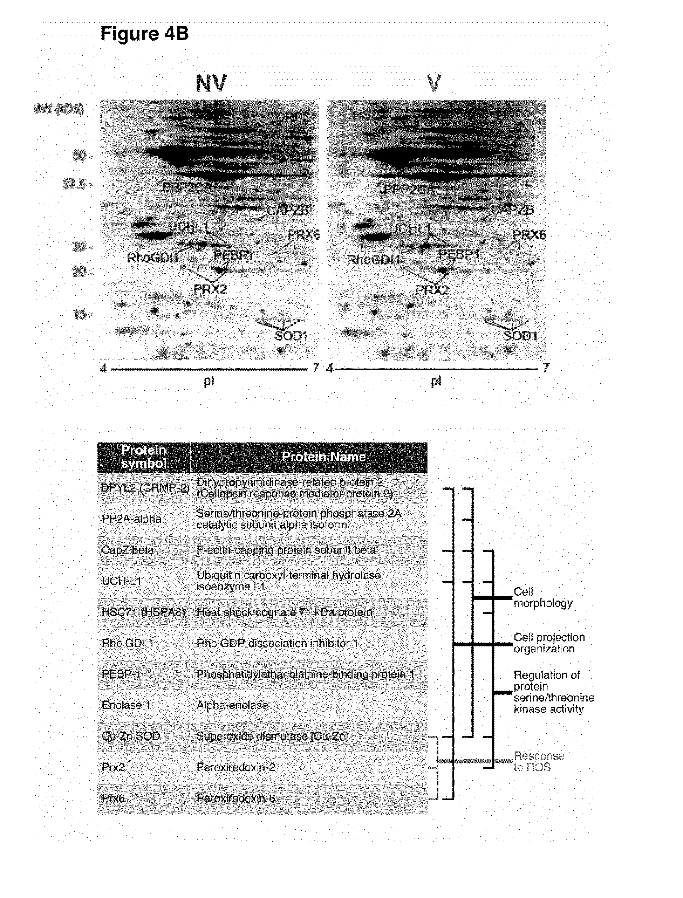

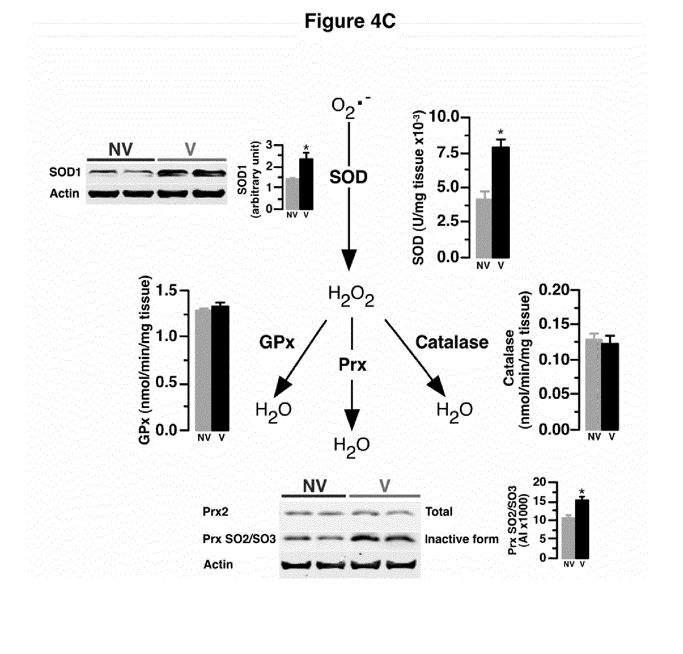

Figures 4A, 4B and 4C shows that oxidative stress is involved in vulnerability to depression. The experiments concerned in figures 4A, 4B and 4C are carried out on 31 days after social defeat (see Figure 10).
   Figure 4A: The activity of aconitase decreased in response to redox challenge in animals classified as vulnerable (V) as compared to non-vulnerable (NV), 31 days after the end of the social defeat procedure (D31). Redox imbalance results in lipid oxidative damage (increase in lipid peroxidation, MDA) in V animals (NV: n = 7-8; V: n = 6-8).
   Figure 4B: Differential protein profiles on two-dimensional gel electrophoresis in hippocampus of NV and V rats and identification of protein spots by mass spectrometry. In silico functional analysis of the dataset with GO Term Finder (Boyle et al., Bioinformatics, 20, 3710 [2004]) indicated four main functional categories including redox-related proteins involved in ROS response (n=4 per group, performed twice).
   Figure C: The quantity and activity of superoxide dismutase (SOD), which catalyzes hydrogen peroxide (H2O2) production, were greater in V than in NV animals (NV: n = 8; V: n = 6). Peroxiredoxin2 (Prx2), involved in the reduction of H2O2 to water, is present mostly in its inactive form, Prx-sulfinic/sulfonic (SO2/SO3) in V animals, with no change to glutathione peroxidase (GPx) and catalase activities (NV: n = 6-8; V: n = 5-6). The results of statistical analyses are presented in Table 2A. * P < 0.05 vs NV animals.

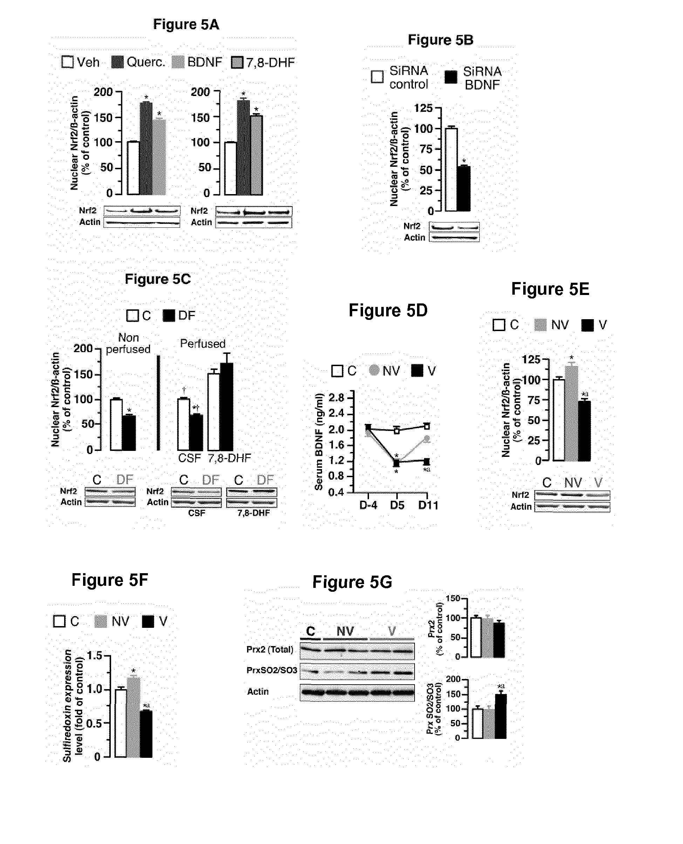

Figures 5A, 5B, 5C, 5D, 5E, 5F and 5G show that BDNF controls the redox-sensitive Nrf2 pathway. The experiments concerned in figures 5D, 5E, 5F and 5G are carried out on 11 days after social defeat (see Figure 10). Figure 5A: Incubation with BDNF (100 ng/ml) or 7,8-dihydroxyflavone (7,8-DHF, 10 µM) induced a marked increase in the nuclear Nrf2 translocation in the hippocampal HT22 cells. The action of BDNF and 7,8-DHF is similar to that of the well known natural Nrf2 activator, quercetin (Querc., 10 µM) (Hur and Gray, Curr Opin Chem Biol 15, 162 [2011]) (n = 4). Figure 5B: BDNF knockdown by a siRNA strategy resulted in lower levels of Nrf2 translocation to the nucleus than transfection with a control siRNA, in HT22 hippocampal cells.
   Figure 5C: Hippocampi extracted from animals 5 days after a chronic social stress (DF) had lower levels of Nrf2 in the nucleus than control hippocampi (C: n = 6, DF: n = 6). In C and DF animals, hippocampal perfusion with CSF enriched with 7,8-DHF (10 µM) induced a marked translocation of Nrf2 to the nuclear compartment (C; n = 6-7; DF; n = 6-7).
   Figure 5D: 11 days after DF (D11), non-vulnerable (NV) and vulnerable (V) animals can be distinguished on their serum BDNF levels.
   Figure 5E: Nuclear Nrf2 levels remained low only in V rats and were significantly higher in the NV animals than in the control (C) group (C: n = 7, NV: n = 7; V: n = 6).
   Figure 5F: The functional activity of Nrf2 was modified, as shown by the levels of sulfiredoxin mRNA decreased in V animals and increased in NV animals (C: n = 7, NV: n = 7; V: n = 6).
   Figure 5G: In V animals, peroxiredoxin 2 (Prx2) was inactivated, being converted to Prx-sulfinic/sulfonic (SO2/SO3) (n = 5; NV: n = 6; V: n = 5). The results of statistical analyses are presented in Table 3B1- 4. * P < 0.05 vs vehicle (Veh), vs siRNA control, or vs C animals; a P < 0.05 vs NV group; † P < 0.05 vs the corresponding 7,8-DHF-treated group.
Figures 6A, 6B, 6C and 6D show BDNF, the Nrf2/Keap1 system and oxidative stress in non-perfused and perfused hippocampi of post stress animals. The experiments concerned in figures 6A, 6B, 6C, 6D are carried out on 5 days after social defeat (see figure 10).

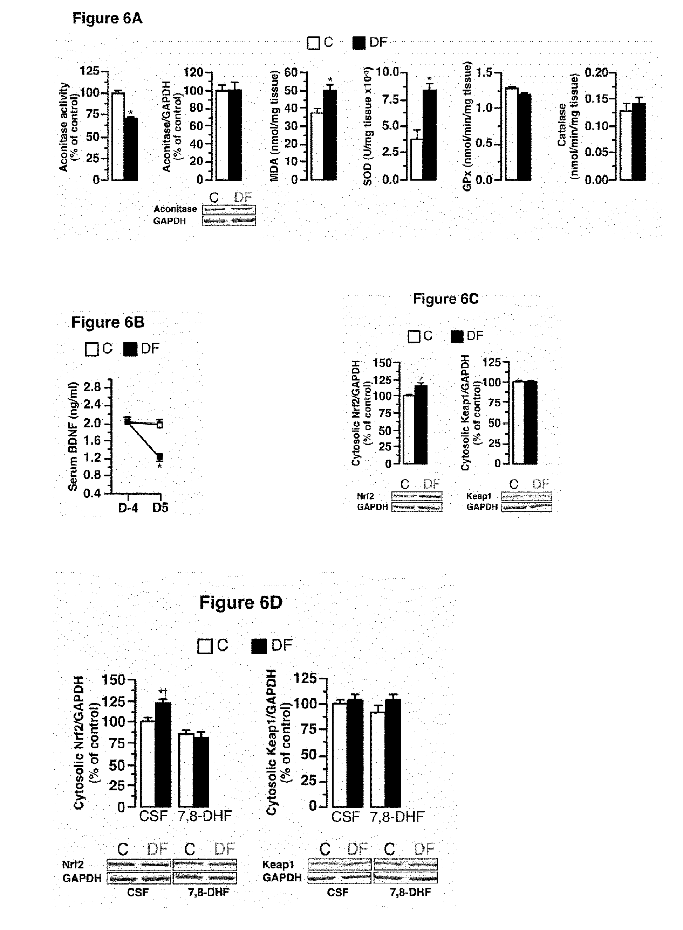

   Figure 6A: Five days after the chronic social defeat procedure (D5), defeated animals (DF) displayed oxidative stress, as shown by the decrease in aconitase activity with no change in the amount of this enzyme, the high level of lipid peroxidation (MDA), the increase in superoxide dismutase activity (SOD) and the lack of change in glutathione peroxidase (GPx) and catalase activities (C: n = 6-9; DF: n = 9-12).
   Figure 6B: At D5, all animals (DF) had lower serum BDNF levels than control (C) animals. Figure 6C: Nrf2 and Keap 1 levels in non perfused hippocampi of defeated (DF) and control rats. The hippocampi of DF animals displayed an increase in cytosolic Nrf2 concentration with no change in Keap1 levels (C: n = 6, DF: n = 6).
   Figure 6D: Nrf2 and keap 1 levels in perfused hippocampi of defeated (DF) and control rats. The hippocampi of DF and control (C) rats were perfused with artificial cerebrospinal fluid (CSF) or with artificial CSF enriched with 7,8-dihydroxyflavone (7,8-DHF; 10 µM). 7,8-DHF perfusion (10 µM) induced a decrease in cytosolic Nrf2 concentration in DF animals, and a tendency for this concentration to decrease in control (C) animals with no change in Keap1 concentration (C; n = 6-7; DF; n = 6-7). The results of statistical analyses are presented in Table 4 B1-3. * P < 0.05 vs C animals; † P < 0.05 vs the corresponding 7,8-DHF-treated group.

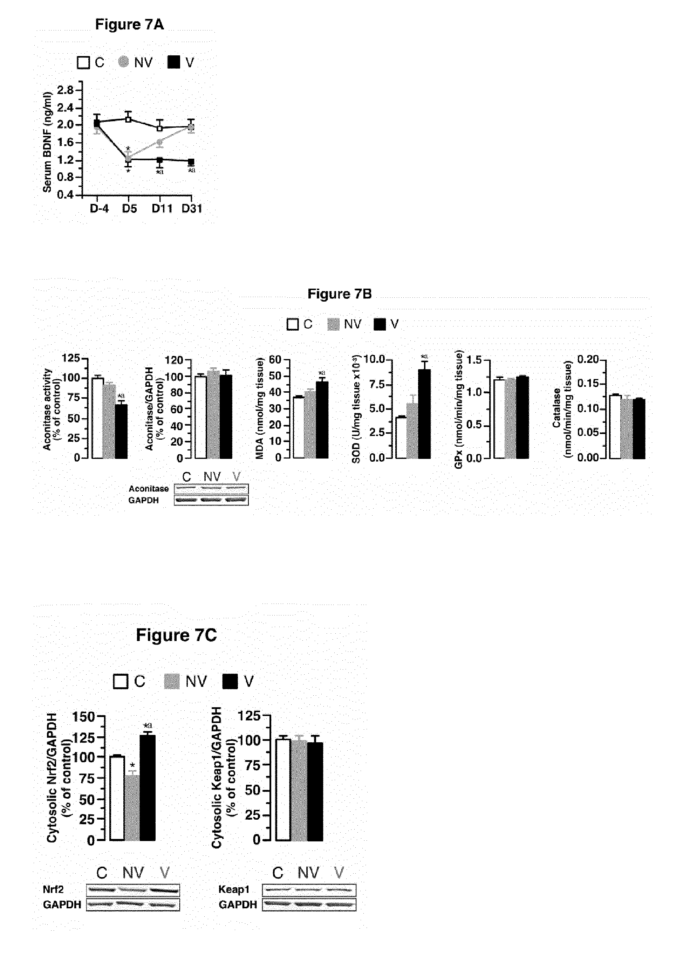

Figures 7A, 7B and 7C show oxidative stress in vulnerable, non vulnerable and control animals at D11.
   Figure 7A: Longitudinal follow-up of BDNF levels. Animals identified as V at D11 remained V at D31.
   Figure 7B: At the key time point, 11 days after the chronic stress, when non vulnerable (NV) and vulnerable (V) rats were identified, oxidative stress persisted only in V animals (persistent increase in SOD activity and lipid peroxidation [MDA] and a decrease in aconitase activity). In animals in which serum BDNF concentrations returned to control values (NV animals), oxidative stress disappeared. The amount of aconitase and the activity of the two enzymes (GPx; catalase) were similar in V (n = 6), NV (n = 6-8) and control (C; n = 6) animals. The experiments concerned in figure 7B are carried out on 11 days after social defeat (see figure 10).
   Figure 7C: The cytosolic Nrf2 concentration remained high in V rats only and was significantly lower in NV animals than in the control (C) group, with no change in Keap1 levels (C: n = 7, NV: n = 7; V: n = 6). (C). The results of statistical analyses are presented in Table 5 C1,2. * P < 0.05 vs C animals; a P < 0.05 vs NV animals. The experiments concerned in figure 7C are carried out on 11 days after social defeat (see figure 10).

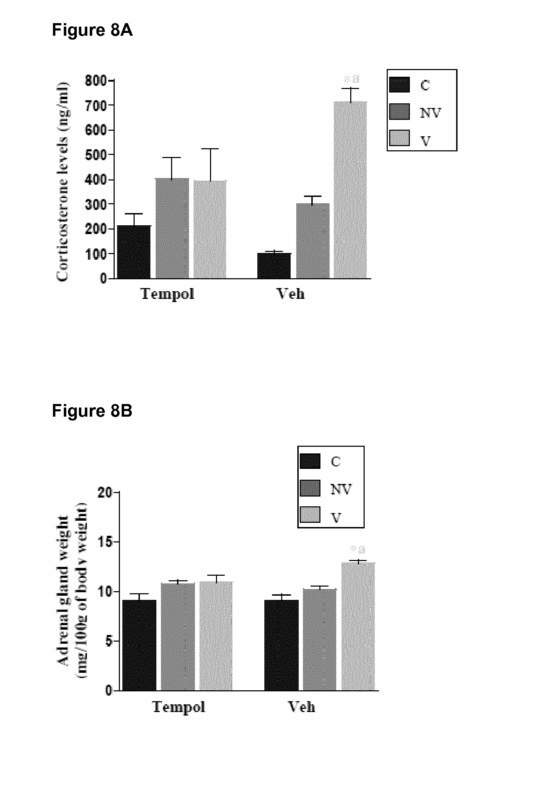

Figure 8A and 8B show the effect of TEMPOL on corticotropic axis activity in the control (C) group, non-vulnerable group (NV) or vulnerable group (V). A pharmaceutical vehicle (Veh) as control is also administrated in C, NV or V groups (C + Tempol : n = 6 ; NV + Tempol : n = 9 ; V + Tempol : n = 7 ; T + Veh. : n = 7 ; NV + Veh. : n = 7 ; V + Veh. : n = 8). The results of statistical analyses are presented in Table 6. * P < 0.05 vs C group treated with Veh.; a P < 0.05 vs NV group treated with Veh., † P < 0.05 vs the corresponding tempol-treated group. The corticotropic axis activity is determined by corticosterone levels (Figure 8A) and adrenal gland weight (Figure 8B).
Figure 9 illustrates the effect of TEMPOL on serum BDNF levels in the control (C) group, non-vulnerable group (NV) or vulnerable group (V). A pharmaceutical vehicle (Veh) as control is also administrated in C, NV or V groups (C + Tempol : n = 10 ; NV + Tempol : n = 10 ; V + Tempol : n = 9 ; C + Veh. : n = 16 ; NV + Veh.: n = 19 ; V + Veh.: n = 14). The results of statistical analyses are presented in Table 7. *P <0.05 vs basal levels.
Figure 10 is a summary of social defeat and chronic mild stress experimental design and displays the relation between serum BDNF levels depressive profile in vulnerable population and non-vulnerable population.

### Examples

### 1. Materials and methods

### Animals

Male Sprague-Dawley rats, weighing 290-310 g, were obtained from the same breeder (Centre d'Elevage R. Janvier, 53940 Le Genest-St-Isle, France) and used as intruder rats. On their arrival at the laboratory, they were housed in chronobiologic animal facilities (Enceinte Autonome d'Animalerie, A110SP, Thermo Electron Corporation, Saint Herblain, France) equipped with regularly spaced, sound-proof, controlled-temperature compartments, each supplied with filtered air. Sprague-Dawley rats were housed together for four days and were then transferred to individual cages (1: 45 cm; w: 25 cm; h: 17 cm) 14 days before the start of the experiments. Male wild-type Groningen (WTG) rats were used as resident rats in confrontation encounters (de Boer et al., 2003). All animals were kept under controlled environmental conditions (22 ± 1°C, 60% relative humidity, 12/12 h light-dark cycle with lights on at 07:00, food and water ad libitum). Procedures involving animals and their care were performed in accordance with institutional guidelines conforming to national and international laws and policies (Council directive #87-848, October 19, 1987, Ministère de l'Agriculture et de la Forêt, Service Vétérinaire de la Santé et de la Protection Animale, authorizations #75-1178 to J.J.B.). All measurements were performed by individuals blind to treatment group.

### Social-defeat procedure

The social-defeat procedure (CSD) (D-3 to D0) was performed as previously described (see Becker et al., 2001; Blugeot et al., 2011).

Briefly, this procedure involved subjecting the same pairs of residents and intruders to four daily conditioning sessions. The 45-min conditioning sessions were divided into two consecutive periods. During period I (30 min), intruders were placed individually in a protective cage within the resident animal's home cage. The protective cage allowed unrestricted visual, auditory, and olfactory contact with the resident but prevented close physical contact. During period II (15 min), the protective cage was removed, either with the resident remaining present, allowing physical confrontation with the intruder (3 to 4 confrontations of ∼10 s, during each of which the intruding (defeated) animal was always dominated by the resident rat) or with the resident removed, giving the intruder access to the entire resident home cage (control intruders). The control intruders were therefore never physically attacked and defeated by the resident.

### Serum BDNF assay

Blood samples (200 µl) were collected from conscious rats at various time points (D-4, D5, D11, D31 at midday). These samples were taken from the tail vein and were collected in Eppendorf tubes. The samples were centrifuged to separate off the serum, which was stored at -20°C until BDNF analysis. BDNF concentrations were determined at a dilution of 1:25, with a commercial BDNF assay (Promega Corporation), in 96-well plates (Coming Costar® EIA plate), according to the manufacturer's instructions.

### Hippocampal BDNF

When the rats were killed, the brains were rapidly removed. Bilateral hippocampi were rapidly dissected and stored at 80°C. At the time of analysis, samples were weighed, and BDNF was extracted as described by Szapacs et al. (2004, J Neurosci Methods 140:81-92). Two milliliters of lysis buffer (100 mM PIPES, 500 mM NaCl, 0.2% Triton X-100, 0.1% NaN3, 2% BSA, and 2 mM EDTA) containing freshly prepared protease inhibitors (200 µM PMSF, 0.3µM aprotinin, and 10 µM leupeptin) were added to each sample. Samples were then sonicated by pulses at 1 s intervals for 15 s. An additional 1 ml of lysis buffer was added, and the samples were resonicated. All homogenates were centrifuged at 16,000 xg for 30 min at 4°C, and supernatants were removed and frozen at 20°C until assay. BDNF concentrations were determined at dilution of 1:10 with the commercial BDNF assay described above.

### Protein profiling and identification

Hippocampi were homogenized with a glass homogenizer in a urea-based solubilization buffer containing 8 M urea, 2 M thiourea, 4% 3-(3-chloramidopropyl) dimethylammonio-1-propanesulfonate (CHAPS), 20 mM dithiothreitol, 1.2 M spermine and protease inhibitor cocktail. Homogenates were clarified by centrifugation (15,000 x g, 15 min) and stored at -80°C. Before application, all samples were diluted in solubilization buffer supplemented with 1% ampholites. Samples (125 µg protein loaded) were applied by active rehydration (50 V, 10 h) to IPG strips (pH 4-7, Biorad, France) and focused in PROTEAN IEF Cells (Bio-Rad) for a total of ∼16 kVh. After isoelectric focusing (IEF), the strips were immediately equilibrated for 2 x 10 min with 50 mM Tris-HCl, pH 8.8, in 6 M urea, 30% glycerol and 2% SDS. DTT (2%) was included in the first and iodoacetamide (2.5%) in the second equilibration. The second dimension was SDS-PAGE in a 12 % polyacrylamide gel. Proteins were stained with Coomassie blue G250 and gels were imaged with the Odyssey® infrared imaging system and analyzed with ImageMaster 2D Elite software, version 4.01 (Amersham Biosciences). Spots displaying differential expression were excised from the gels and subjected to a standard in-gel tryptic digestion procedure, as described elsewhere (Brouillard et al., 2005), except that the reduction and alkylation steps were omitted. For MALDI-TOF MS analysis, peptide powders were resuspended in 0.5% trifluoroacetic acid, spotted onto a MALDI target, mixed in equal volumes with matrix (10 mg/ml -cyano-4-hydroxycinnamic acid in 70% ACN 0.1% TFA), and then analyzed with an AB/Sciex 5800 TOF/TOF mass spectrometer. An external calibration was performed with standard peptide solutions Cal Mix1 and Cal Mix2 (Applied Biosystems). Monoisotopic peptide mass values extracted by Data Explorer 4.9 software (Applied Biosystems) were used for protein identification, based on searches against the UniProtKB/Swiss-Prot database with the on-line Mascot search engine (www.matrixscience.com). The searches were conducted with the following settings: trypsin as the digestion enzyme, one missed cleavage allowed, 30 ppm tolerance, carbamidomethyl as a fixed modification and methionine oxidation as a variable modification. The criterion used for positive identification was a statistically significant Mowse score (P<0.05).

### Redox parameters

The animals were decapitated and the left hippocampus was homogenized in ice-cold buffer (20 mM Hepes pH 7.2, 1 mM EGTA, 210 mM mannitol, 70 mM sucrose). Homogenates were centrifuged and the supernatants were retained for analysis. Catalase and total SOD activities were immediately determined with commercially available kits (Cayman Chemical, Ann Arbor, MI, USA), in accordance with the manufacturer's instructions. SOD and catalase specific activities are expressed as units per mg of hippocampus and as nmoles of formaldehyde formed per minute per mg of hippocampal tissue, respectively.

The right hippocampus was homogenized in ice-cold buffer (Tris-HCl 50 mM pH 7.5, 150 mM NaCl, 1 mM EDTA) and used for TBARS and glutathione peroxidase (GPx) assays.

For the TBARS assay, protease inhibitors (50 µl/ml), DOC (1 %), SDS (0.1 %) and Triton® X-100 (0.5 %) were added immediately in ice-cold buffer. For the GPx assay, 0.1 M dithiothreitol was added immediately in ice-cold buffer. The homogenates were centrifuged and the supernatants were retained for analysis. Lipid peroxidation was assessed by measuring TBARS formation with a commercial assay kit (Cayman Chemical, Ann Arbor, MI, USA), in accordance with the manufacturer's instructions. TBARS concentration was calculated from an MDA standard curve and normalized. Total GPx activity in the homogenate was measured in accordance with the kit manufacturer's instructions (Cayman Chemical, Ann Arbor, MI, USA). GPx specific activity is expressed as nmoles of NADPH consumed per minute per mg of hippocampal tissue. No difference in hippocampal weight was observed between defeated, vulnerable, non-vulnerable and control animals.

### Aconitase activity

Rats were killed by decapitation and their right hippocampi were rapidly removed and homogenized in ice-cold buffer (5 mM Tris-HCl pH 7.5, 210 mM mannitol, 70 mM sucrose, 1 mM EDTA and 2.3 mM sodium citrate). Homogenates were rapidly centrifuged (14,000 x g, 10 min, 4°C) and the pellets were immediately frozen in liquid nitrogen and stored at -80°C. Aconitase activity was measured spectrophotometrically (Gardner et al., 1994), by monitoring the linear absorbance change at 340 nm, at 25°C, in a 1.0-ml reaction mixture containing 50 mM Tris HCl (pH 7.5), 0.05% BSA, 2 mM sodium citrate, 2 mM MnC12, 0.4 mM NADP+, 0.85 units of isocitrate dehydrogenase, and 10-20 µg of protein extract. The absorbance change associated with aconitase activity was linear with respect to time and protein concentration. One milliunit of aconitase activity was defined as the amount of enzyme catalyzing the formation of 1 nmol of D-isocitrate per min and per mg.

### Nuclear and cytosolic extracts

Hippocampi were homogenized in ice-cold buffer A (10 mM Hepes, 150 mM NaCl, 1 mM EDTA, protease inhibitor cocktail [5 µl/ml] and phosphatase inhibitor cocktail [10 µl/ml]) and Nonidet P-40 (0.5 %) was then added. After 10 min on ice, homogenates were centrifuged (1,200 x g, 10 min, 4°C). Supernatants were saved as the cytoplasmic fraction. Pellets were washed in ice-cold buffer A and sedimented by centrifugation at 1,200 x g for 10 min at 4°C. Nuclear yield and integrity were checked by Giemsa staining and microscopy. Nuclei were resuspended in ice-cold buffer B (20 mM Hepes, 420 mM NaCl, 1.2 mM MgC12, 0.2 mM EDTA, 25 % glycerol, 0.5 mM DTT, protease inhibitor cocktail [5 µl/ml] and phosphatase inhibitor cocktail [10 µl/ml]). Samples were sonicated (15 s, 4°C) and vortexed every 5 min for 20 min. The cytoplasmic and nuclear fractions were centrifuged at 20,000 x g, for 30 min at 4°C. Supernatants were rapidly frozen in liquid nitrogen and stored at -80°C. A similar protocol was used for cell nuclear and cytosolic extracts.

### Western blotting

40 µg of nuclear or cytosolic protein (or 20 µg for cell experiments) was resolved by SDS-polyacrylamide gel electrophoresis in a 12% polyacrylamide gel. The bands were transferred to nitrocellulose or PVDF membranes (Invitrogen, France) for immunoblotting according to standard procedures. We used the following antibody dilutions: 1:250 for rabbit polyclonal antibodies against Nrf2 (Santa Cruz Biotechnology, France), 1:500 for rabbit polyclonal antibodies against Keap1 (Santa Cruz Biotechnology) and 1:5000 for rabbit polyclonal antibodies against aconitase 2 (Gene Tex, France). A dilution of 1:10,000 was used for the fluorescent IRDye® 800CW secondary antibody (ScienceTec, Paris, France). Membranes were also probed with a mouse monoclonal antibody against GAPDH or a mouse monoclonal antibody against beta-actin (dilutions of 1:10,000 and 1:1,000, respectively, Sigma-Aldrich, France) and with the IRDye® 680CW secondary antibody from ScienceTec (1:10,000), to correct for protein loading. Images were acquired with the Odyssey® infrared imaging system (LI-COR Biosciences). Protein bands were quantified with Odyssey® software (version 3.0, LI-COR Biosciences). For SOD1, Prx2 and PrxSO2/SO3 analysis, we used the following antibody dilutions: 1:1000 for rabbit polyclonal antibodies against SOD (Euromedex), 1:2000 for rabbit polyclonal antibodies against PeroxiredoxinSO2/SO3 (Abcam), 1:1000 for rabbit polyclonal antibodies against Peroxiredoxin2 (Abcam). Horseradish peroxidase-conjugated secondary antibodies were used and membranes were developed with the ECL Plus detection reagent (Pierce, Rockford, IL). Images were acquired with a digital camera-based imaging system (ChemiDoc, Biorad) and analyzed with Quantity One software (Bio-Rad).

### Real Time-qPCR

* RNA extraction: Total RNA was isolated from frozen hippocampi with a NucleoSpin RNA II Purification kit, according to the manufacturer's recommendations (Macherey- Nagel, France). RNA quality and concentration were evaluated from absorbance measurements with a NanoDrop spectrometer (Thermo Fisher Scientific, Labtech, France).
* RT-qPCR. For real-time PCR analysis, first-strand cDNA synthesis (0.6 µg of total RNA per 20 µl reaction) was performed with a High-Capacity cDNA Reverse Transcription kit (Applied Biosystems, France). Real-time PCR amplification of each sample was performed in triplicate, on an ABI Prism 7300 (Applied Biosystems) with the ABgene Absolute QPCR ROX Mix (ABgene). Assay-on-Demand GeneTaqManPCR probes (Applied Biosystems) were used for target genes: sulfiredoxin (Rn01536084-g1*) and glyceraldehyde-3-phosphate dehydrogenase (GAPDH) (Rn99999916-S1). The difference in mean threshold cycle (ΔCₜ) values was determined and normalized with respect to expression of the housekeeping gene (GAPDH). The other hippocampus dissected from the same animal was used for western blot analysis.

### Hippocampal HT-22 cells

Cells were maintained at 37°C, under an atmosphere containing 5% CO2, in Dulbecco's modified Eagle medium (DMEM) supplemented with 10% fetal bovine serum and 1% penicillin /streptomycin. In some experiments, medium (without serum) was enriched in BDNF (100 ng/ml), 7,8-dihydroxyflavone (10 µM) or quercetin (10 µM) for 30 min, cells were then subjected to subcellular fractionation to obtain nuclear and cytosolic extracts.

### Cell culture and siRNA Knockdown

BDNF knockdown was performed using Silencer® Select siRNA targeting BDNF or a non-targeting Silencer® Select siRNA as control. siRNA transfections were done at a final concentration of 50 nM by using Lipofectamine RNAiMAX (Invitrogen) according to the manufacturer's forward protocol. Briefly, 16 µL of lipofectamine RNAiMAX were combined with 400 pmol siRNA in 800 µl of opti-MEM to transfect 2.5 × 106 cells plated onto 75 cm2 flasks. Cells were cultured in DMEM without serum and harvested 48 h post-transfection.

### Golgi staining

At D11 and D31, unperfused brains were rapidly removed and processed with the FD Rapid Golgistain kit (FD NeuroTechnologies), as previously described by Blugeot et al. (2011). Histological quantification: for each rat, the total dendrite length of apical CA3 dendritric branches was measured for selected neurons with AxioVision 4.7 software and a Zeiss Imager M1 microscope. 3-5 hippocampal neurons per rat are selected for measurement by an investigator blind to the origin of the experimental animal. Dendritic tracing was quantified by Sholl analysis (Sholl, 1953). The number of intersections between the dendrites and the concentric circles was counted and plotted as a function of the distance from the soma. The density of dendritic spines was estimated by counting the number of apical dendritic spines in 3-4 segments of 15 µm located in the stratum radiatum, at 1000x resolution. Spine density was expressed as the number of spines per 15 µm length of apical dendrite.

### Sensitization paradigm (SSP)

Sprague-Dawley rats were subjected to chronic social defeat (CSD) and then, four weeks later, to three weeks of chronic mild stress (SSP rats). The chronic mild stress protocol (CMS) was performed as previously described (see Blugeot et al., 2011). The control rats were control intruders (not subjected to CSD) not exposed to the three weeks of CMS (C rats). The experimental design is summarized in figure 10.

### Forced swimming test (FST)

Four days after the end of SSP paradigm (D52), FST experiments were performed between 08:00 and 11:30. Experiments were performed as described by Becker et al. (2008, *Mol. Psychiatry* 13, 1079) and Blugeot et al. (2011). Immobility time was measured with a stopwatch. A rat was considered immobile when floating and making only the movements necessary to keep its nostrils above the surface of the water. A trained experimenter blind to the treatment observed the animals and measured immobility time. The temperatures of both the room and the water were checked at the end of each session. Each rat was subjected to only one swimming session.

### Sweet water consumption

Experiments were performed as described by Becker et al. (2008) and Blugeot et al. (2011). Sucrose and water intakes were measured daily at 9:00 a.m. Weekly sweet water consumption was expressed as a percentage of control values (measured one week before the beginning of the conditioning sessions). Sweet water consumption indicated a preference for sucrose. During the control period, rats drank significantly more sweet water (≈ 30 ml/day) than plain water (≈ 15 ml/day), thus displaying a preference for sucrose. Plain water intake did not change [≈ 15 ml/day]) when sweet water consumption decreased.

### Drugs and treatments

Tempol (4-hydroxy-2,2,6,6-tetramethylpiperidine 1-oxyl), which was administered at a dose of 288 µmol/kg/day [Sigma-Aldrich, St Louis, MO, USA]), was dissolved in distilled water and delivered via ALZET osmotic mini-pumps (2002, Charles River Laboratories, France). Tempol- or distilled water-filled pumps were implanted subcutaneously on the back of intruder rats under light anesthesia with isofluorane, 11 days after completion of the social-defeat procedure (D11), and were left in place for 15 days.

### Statistical analyses

Differences in SOD activity, TBARS, aconitase activity or quantity, glutathione peroxidase activity, catalase activity, apical dendrite length of CA3 neurons and hippocampal CA3 dendritic spines were validated by one-way analysis of variance (ANOVA). Serum BDNF concentration was analyzed by one-way ANOVA for repeated measures. The stress effect and treatment effect were validated by two-way ANOVAs. All data are presented as means ± SEM. If ANOVA revealed a significant effect, a post-hoc Bonferroni test was carried out to determine whether differences were truly statistically significant.

### Superfusion of the hippocampus

The hippocampus was dissected and cut into 8 pieces, placed in the tissue chamber for perfusion and suspended in an artificial cerebrospinal fluid (aCSF, 1x) consisting of a 1x solution (136 mM NaCl, 16.2 mM NaHCO3, 5.4 mM KCl, 1.2 mM NaH2PO4, 2.2 mM CaC12, 1.2 mM MgC12 and 5 mM glucose) or in 1x aCSF containing 7,8-DHF (10-5 M). The pH of the solutions was adjusted to 7.3 by bubbling with an O2/CO2 mixture (95.5%/4.5%). The superfusion was equally dispersed into 8 thermostatically controlled (37°C) chambers at a flow rate of 1 ml/4 min (Benoliel et al., 1992). After 1 h, the tissue was collected and subjected to subcellular fractionation to obtain nuclear and cytosolic extracts.

### 2. Results

### Oxidative stress is involved in vulnerability to depression

The level of ROS which reflects oxidative stress in an individual is determined by the measurement of intracellular aconitase activity, which is highly sensitive to ROS, in animals with low serum BDNF levels (V group) and normal serum BDNF level (NV group) 31 days after CSD (D31). V animals presented an oxidative stress as demonstrated by decreased aconitase activity levels, despite similar protein levels -reflecting ROS increase-, and lipid peroxidation (MDA) (Fig.4A).

A proteomic analysis is used to identify the redox-related proteins involved in such redox imbalance. Two-dimensional electrophoresis protein profiling showed differences in expression for several abundant protein spots between NV and V animals, leading to the identification of 11 unique proteins by mass spectrometry (Fig.4B).

Among these, Cu-Zn superoxide dismutase (Cu-Zn SOD) and peroxiredoxins (Prxs) constitute a homogeneous functional subset of enzymes directly involved in the regulation of ROS, including superoxide (O2•-) and hydrogen peroxide (H₂O₂). Cytosolic Cu-Zn SOD is an inducible enzyme (Wassmann S. et al., Hypertension, 44, 381 [2004]) that catalyzes H₂O₂ production from O2•- (Fig.4C). Since high H₂O₂ concentrations are deleterious, detoxifying enzymes, such as peroxiredoxins (Prxs), catalase and glutathione peroxidases (GPxs) reduce H₂O₂ into water (Fig.4C). Thus, they, limit H₂O₂ accumulation and its subsequent conversion to the highly noxious hydroxyl radical responsible of oxidative damage to macromolecules, including rounds of lipid peroxidation. In keeping with increased level of oxidative stress (Bilici et al., J. Affect. Disord. 64,43 (2001)), both the quantity and activity of SOD were increased in V as compared to NV animals (Fig.4C), leading to higher H₂O₂ production which needs to be reduced. Although GPxs and catalase levels were not affected, Prx2 was overoxidized in V animals (Fig.4C). Prx2, a tightly regulated enzyme strongly expressed in hippocampal neurons, uses a redox-active peroxidatic cysteine to reduce H₂O₂, resulting in the formation of a cysteine sulfonic acid (Cys-SOH), which is then reduced by disulfide bond formation (Bell and Hardingham, Antioxid. Redox. Signal 14,1467 (2011)). Catalytic inactivation of Prx2 by hyperoxidation results in the formation of a sulfinic acid form (Prx-SO2H), preventing sulfide bond formation (Phalen et al., J. Cell. Biol. 175,779 (2006)), favoring thus H₂O₂ accumulation (Woo et al., Science 300,653 (2003)). The decreased aconitase activity and increased lipid peroxidation in V animals is consistent with a Prx2-dependent accumulation of H₂O₂.

### BDNF controls the redox-sensitive Nrf2 pathway

Hippocampal HT-22 cells were used to prove that BDNF can control bi-directionally Nrf2 translocation. Firstly, it was confirmed that the natural Nrf2 activator quercetin induces a translocation of Nrf2 to the nucleus (Fig.5A), and a corresponding decreased Nrf2 cytosolic fraction. Enriching the milieu with BDNF or with 7,8-dihydroxyflavone (7,8-DHF), defined as a selective TrkB receptor agonist (Jang et al., Proc. Natl. Acad. Sci. U.S.A. 107,2687 (2010)), produced similar effects on Nrf2 (Fig.5A), while Keap1 levels were not affected. Since HT-22 cells lack TrkB receptors (Rössler et al., J. Neurochem. 88,1240 (2004)), Nrf2 translocation is most likely due to an intracellular action of 7,8-DHF, and most likely BDNF, after their internalization. BDNF knockdown with siRNA decreased by nearly 50% Nrf2 translocation (Fig.5B) resulting in Nrf2 accumulation in the cytosol. This suggests that the cytosolic fraction of BDNF exerts a tonic translocation of Nrf2 to the nucleus to produce a basal level of antioxidants.

It is confirmed that the same mechanism is operant in the experimental model of vulnerability to depression. Five days following CSD (D5), all defeated animals were characterized by oxidative stress (Fig. 6A), consistent with the antioxidant alteration in various models of stress, by low BDNF levels (Fig. 6B) and by decreased nuclear levels of Nrf2 (Fig.5C), associated with higher levels of cytosolic Nrf2 and no change in Keap1 levels (Fig.6C). Perfusing hippocampi with 7,8-DHF induced Nrf2 translocation to the nucleus to the same level in defeated and control animals (Fig.5C), with decrease in cytosolic Nrf2 levels and no change in Keap1 (Fig.6D). Although an involvement of TrkB activation cannot be rule out, the effect of 7,8-DHF perfusion is consistent with its intracellular action on the Nrf2/Keap1 complex found in HT-22 cells. Since serum BDNF levels reflect that of the brain, the results are consistent with the proposal that low levels of BDNF result in decreased Nrf2 translocation, hence failure to activate appropriate anti-oxidant defenses, resulting in oxidative stress.

This scheme was tested at a pivotal time point 11 days after CSD (D11). Longitudinal serum BDNF determinations showed that animals identified as V and NV at D11 remained V and NV until D31 (Fig. 7A). In V animals, BDNF levels and Nrf2 translocation remained decreased as at D5 (Fig.5D,E), and markers of oxidative stress similar to those found at D5 and D31 (Fig.7B). In contrast, oxidative state returned to basal levels in NV animals (Fig.7B), suggesting the activation of defense mechanisms. Accordingly, there was an increase in nuclear Nrf2 translocation (Fig.5E, with a decrease in cytosolic Nrf2, Fig.7C), and subsequently in sulfiredoxin (Fig.5F). Conversely, sulfiredoxin were decreased in V animals (Fig.5F). Whilst Prx2 was overoxidized in V animals, it returned to a normal catalytically active form in NV animals (Fig.5G), most likely via the increase in sulfiredoxin.

Together these results point to the following scheme. The CSD episode results in pro-oxidant conditions, due in part to the inactivation of Prx2 leading to H₂O₂ accumulation. In NV animals, the return to baseline BDNF enables normal Nrf2 function, with the activation of defense mechanisms and a return to normal redox state. In V animals, the continuous low level of BDNF prevents appropriate Nrf2 translocation and activation of defense mechanisms, in particular sulfiredoxin to restore Prx2 activity and H₂O₂ clearance.

### Effect of TEMPOL on oxidative stress state

Chronic treatment with TEMPOL, via anosmotic minipump, began at a key time point (11 days after the chronic stressful event [D11], when vulnerable [V] rats presented an oxidative state and neuroanatomical alterations) for 15 days. Chronic treatment with the antioxidant TEMPOL decreased the oxidative stress in V animals, as shown by the lack of aconitase activity dysfunction, of lipid peroxidation and of SOD dysfunction, with a mild effect on glutathione peroxidase (GPx) activity and no effect on catalase activity (C: n = 5-6; NV: n = 6-8; V: n = 5-6), highlighting the key role of oxidative stress in vulnerability to depression (Figures 1A, 1B, 1C, 1D, 1E).

### Effect of TEMPOL on hippocampal neuron's morphology

Oxidative stress is associated with dendritic retraction and decreased spine density, a characteristic feature of CA3 pyramidal cells in V animals (Fig.2A, 2B). In NV animals, neuroanatomical alterations are restored fully at D31 (Fig. 2A, 2B). Treatment with TEMPOL fully restored dendritic trees and spine density to control levels in both NV and V animals (Fig.2A, 2B). None treated V animals present a depression-like profile in response to three weeks of CMS applied at D31.

### Effect of TEMPOL on depressive phenotype

Figures 3A and 3B show that chronic treatment with the antioxidant TEMPOL, in animals identified as vulnerable at D11 (V, n = 7), prevented the increase in immobility time in the forced swimming test and the decrease in sweet water consumption observed in vehicle-treated V animals after three weeks of chronic mild stress applied 31 days after the end of the CSD protocol (n = 9).

Although TEMPOL was administrated before CMS, the depression-like profile generated by the CMS was abolished (Fig.3A, 3B). Early TEMPOL treatment thus prevented the neuroanatomical changes and the development of a state of vulnerability to depression, confirming that this vulnerability is actually dependent on the residual traces induced by a sustained redox imbalance.

### Statistical analyses

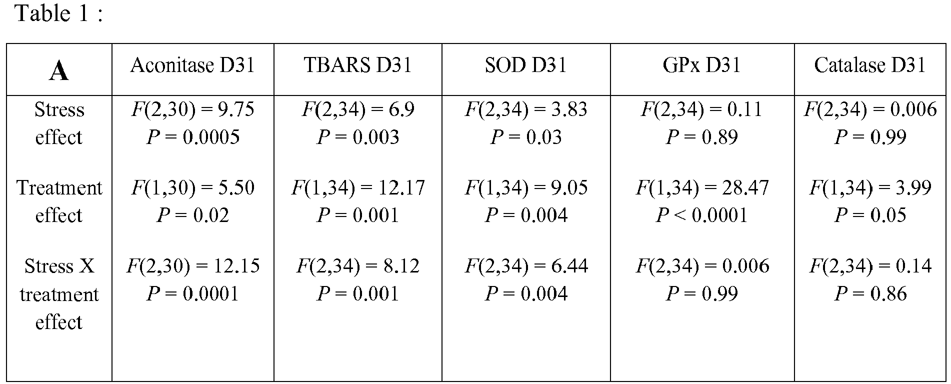

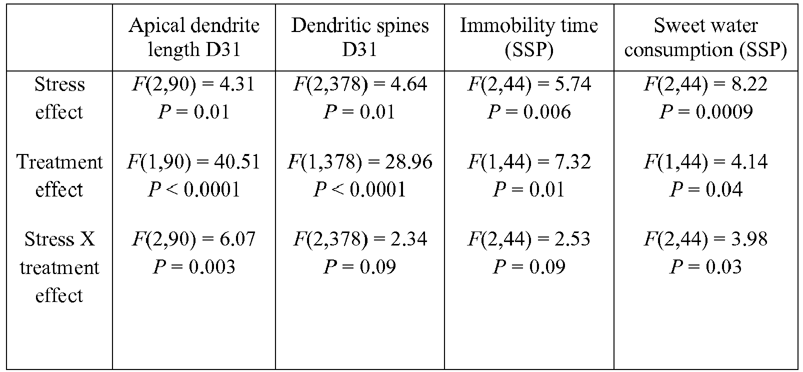

A of table 1 corresponds to statistical analysis of the effects of social defeat stress and of TEMPOL treatment on aconitase, TBARS, SOD, glutathione peroxidase (GPx) and catalase activities, apical dendrite length of CA3 neurons and hippocampal CA3 dendritic spines, based on two-way ANOVA. Statistical analysis of the effects of SSP stress and of TEMPOL on immobility time, sweet water consumption, is based on two-way ANOVA.

**Table 2:**

| **A** | Aconitase D31 (activity) | TBARS D31 | SOD D31 | GPx D31 | Catalase D31 |
|---|---|---|---|---|---|
| Stress effect | *F*(1,13) = 15.82 *P* = 0.0016 | *F*(1,12) = 8.11 *P* = 0.012 | *F*(1,12) = 12.67, *P* = 0.003 | *F*(1,12) = 0.93, *P* = 0.35 | *F*(1,12) = 0.51 *P* = 0.45 |
| | Prx SO2/SO3 | | | | |
| Stress effect | *F*(1,9) = 14.54 *P* = 0.004 | | | | |

A of table 2 corresponds to statistical analysis of the effects of social defeat on aconitase activity, TBARS, SOD, glutathione peroxidase (GPx) and catalase activities and peroxiredoxin (Prx) SO2/SO3 at D31, based on one-way ANOVA.

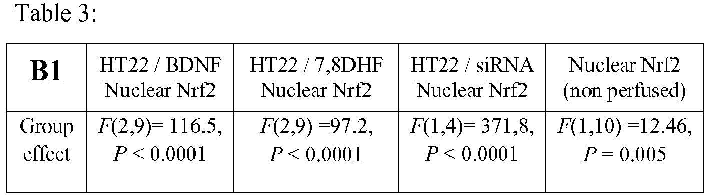

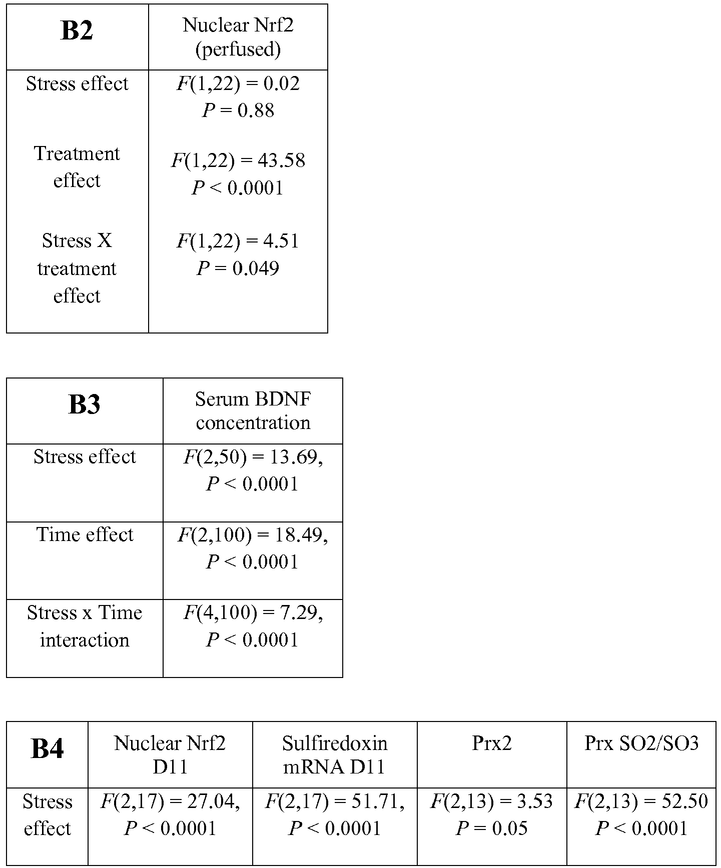

B1 of table 3 corresponds to statistical analysis of the effects of BDNF, 7,8-dihydroxyflavone (DHF) and quercetin or of siRNA BDNF on nuclear Nrf2 concentrations in HT22 cells, based on one-way ANOVA. Statistical analysis of the effects of social defeat on nuclear Nrf2 levels, based on one-way ANOVA.

B2 of table 3 corresponds to statistical analysis of the effects of social defeat stress and of 7,8-DHF perfusion on nuclear Nrf2 levels, based on two-way ANOVA.

B3 of table 3 corresponds to statistical analysis of the effects of social defeat on serum BDNF levels, based on one-way ANOVA for repeated measures.

B4 of table 3 corresponds to statistical analyses of the effects of social defeat stress on nuclear Nrf2, sulfiredoxin gene expression and peroxiredoxin (Prx) at D11, based on one-way ANOVA.

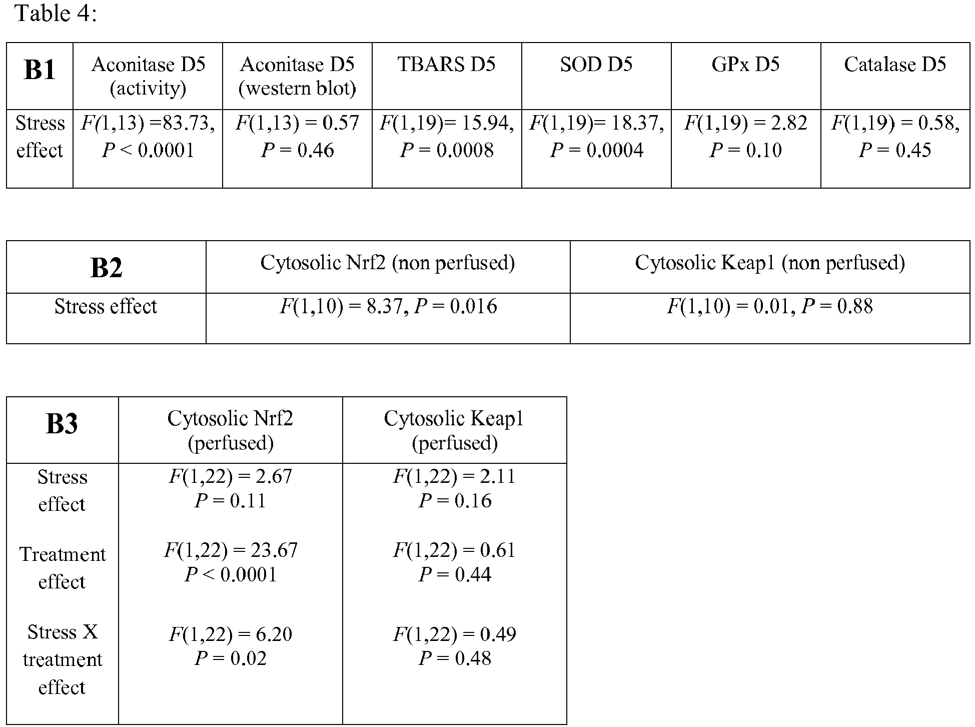

B1 of table 4 corresponds to statistical analysis of the effects of social defeat stress on aconitase (activity and amount), TBARS, SOD, glutathione peroxidase (GPx) and catalase activities at D5, based on one-way ANOVA.

B2 of table 4 corresponds to statistical analysis of the effects of social defeat stress on cytosolic Nrf2 and cytosolic Keap1 levels in non perfused hippocampi, based on one-way ANOVA.

B3 of table 4 corresponds to statistical analysis of the effects of social defeat stress and of 7,8-DHF perfusion on cytosolic Nrf2 and Keap1 levels, based on two-way ANOVA.

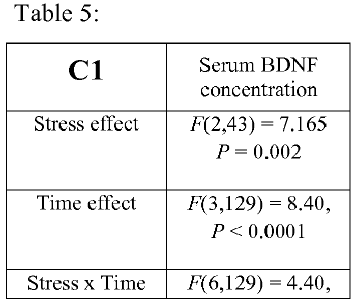

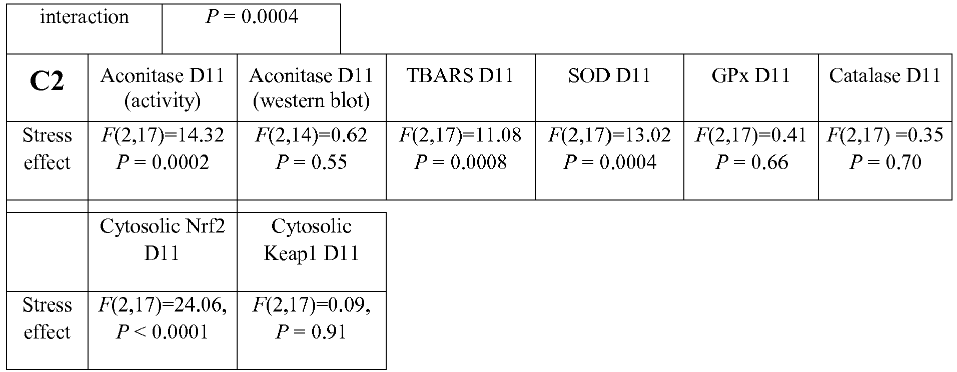

C1 of table 5 corresponds to statistical analysis of the effects of social defeat on serum BDNF levels, based on one-way ANOVA for repeated measures.

C2 of table 5 corresponds to statistical analysis of the effects of social defeat stress on aconitase (activity and amount), TBARS, SOD, glutathione peroxidase (GPx) and catalase activities, cytosolic Nrf2 and cytosolic Keap1 levels at D11, based on one-way ANOVA.

**Table 6:**

| **D** | Corticosterone levels | Adrenal gland weight |
|---|---|---|
| Stress effect | *F*(2,38) = 15.36 *P* < 0.0001 | *F*(2,38) = 16.90 *P* < 0.0001 |
| Treatment effect | *F*(1,38) = 0.34 *P* = 0.55 | *F*(1,38) = 1.28 *P* = 0.26 |
| Stress X treatment effect | *F*(2,38) = 5.78 *P* = 0.006 | *F*(2,38) = 3.34 *P* = 0.04 |

D of table 6 corresponds to statistical analysis of the effects of social defeat stress and of TEMPOL treatment on corticosterone levels and adrenal gland weight, based on two-way ANOVA.

**Table 7:**

| **E** | Serum BDNF concentration |
|---|---|
| Stress effect | *F*(5,72) = 7.43 *P* < 0.0001 |
| Time effect | *F*(3,216) = 11.06, |
| | *P* < 0.0001 |
| Stress x Time interaction | *F*(15,216) = 2.55, *P* = 0.0016 |

D of table 7 corresponds to statistical analysis of the group effects on serum BDNF levels, based on one-way ANOVA for repeated measures.

### Effect of TEMPOL on corticotropic axis activity

TEMPOL and a vehicle have been administrated on control animals, vulnerable animals and non-vulnerable animals. The effect of TEMPOL on coricotropic axis has been evaluated by conticosterone levels (ng/ml) and adrenal gland weight. The results illustrate that the treatment with TEMPOL prevented the increase of corticosterone levels and of adrenal gland weight in vulnerable animals as compared to vulnerable animals treated with vehicle (Figures 8A and 8B).

### Effect of TEMPOL on BDNF level

The treatment with TEMPOL induced the recovery of serum BDNF levels in vulnerable animals as compared to vulnerable animals treated with vehicle (Figure 9)

## Claims

1. Compound having the following formula (I): 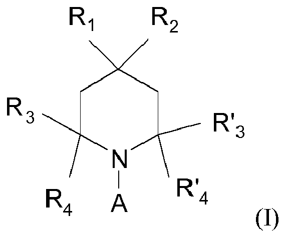 wherein:
- R₁ and R₂ represent independently from each other: H, ORₐ, wherein Rₐ represents H, a C₁-C₁₀-alkyl, aryl or heteroaryl group,
or R₁ = R₂ and represent =O, =NR_{b} wherein R_{b} represents H, a C₁-C₁₀-alkyl, aryl or heteroaryl group, =CR_{c}R_{d}, wherein R_{c} and R_{d} represents independently from each other a C₁-C₁₀-alkyl, aryl or heteroaryl group,
- R₃ and R₄ represents independently form each other H, a C₁-C₁₀-alkyl, aryl or heteroaryl group,
- R'₃ and R'₄ represents independently form each other H, a C₁-C₁₀-alkyl, aryl or heteroaryl group,
- A represents OH or O⁻,
for its use as drug in the treatment and/or the prevention of depressions, with the proviso that said compound is not for use as drug in the treatment of women depressive state associated with premenstrual dysphoric disorder.

2. Compound for its use as drug in the treatment and/or the prevention of depressions according to claim 1, wherein said depressions are resistant to classical treatment.

3. Compound for its use as drug in the treatment and/or the prevention of depressions according to claim 1, wherein said compound is used as drug prescribed as initial treatment of a depression.

4. Compound for its use as drug in the treatment and/or the prevention of depressions according to claim 1 to 3, having the following formula (II): 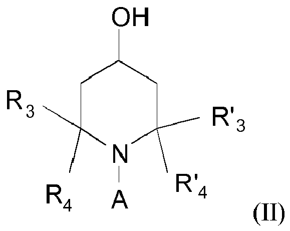 wherein:
A, R₃, R₄, R'₃, and R'₄ are as defined in claim 1.

5. Compound for its use as drug in the treatment and/or the prevention of depressions according to anyone of claims 1 to 4, having the following formula (III): 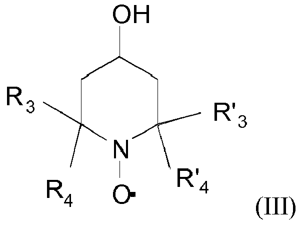 wherein:
R₃, R₄, R'₃, and R'₄ are as defined in claim 1.

6. Compound for its use as drug in the treatment and/or the prevention of depressions according to anyone of claims 1 to 5, having the following formula (IV): 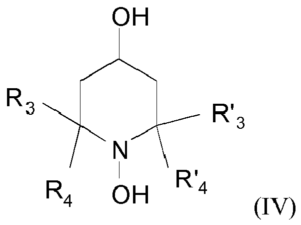 wherein:
R₃, R₄, R'₃, and R'₄ are as defined in claim 1.

7. Compound for its use as drug in the treatment and/or the prevention of depressions according to anyone of claims 1 to 6, having the following formula (V):

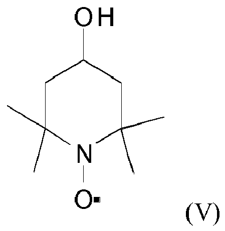

8. Compound for its use as drug in the treatment and/or the prevention of depressions according to anyone of claims 1 to 7, wherein said compound is liable to be chronically administered at a dose comprised 0.1 mg/kg to 300 mg/kg, in particular from 10 mg/kg to 125 mg/kg.

9. Compound for its use as drug in the treatment and/or the prevention of depressions according to anyone of claims 1 to 7, wherein said compound is liable to be acutely administered at a dose comprised from 30 mg/kg to 275 mg/kg.

10. Compound for its use as drug in the treatment and/or the prevention of depressions according to claim 8 or 9, wherein said compound is administered by oral or intravenous route.

11. Compound for its use as drug in the treatment and/or the prevention of depressions according to anyone of claims 1 to 9, said depressions afflicting a human patient, presenting a level of the brain-derived neurotrophic factor decreased in a ratio of about **35%** compared with the one of a control patient, said level being in particular determined in serum, and/or presenting an oxidative stress determined with classical markers, such as the physiological level of catalase and/or the level of lipid peroxidation thiobarbituric acid reactive substances (tBARS) and/or the level of superoxide dismutase (SOD) and/or of lipoperoxidation (as tBARS [Thiobarbituric acid reactive substances], oxysterols) and/or of glutathione reductase (GSR) and/or of the ration GSH/GSSG (reduced glutathione/oxidized glutathione) and/or of protein oxidation and/or of glutathione peroxidase (GPx) and/or of catalase and/or Reactive Oxygen Species (ONOO-, *OH, NO, H₂O₂, O₂⁻) in said patient.

12. Compound for its use in the treatment and/or the prevention of depressions according to anyone of claims 1 to 11, in combination with an compound with antidepressant-like properties or an antidepressant drug, in particular selected from the group consisting of: 7,8 DHF (compound with antidepressant-like properties), SSRI (selective serotonin reuptake inhibitor [as citalopram, escitalopram, fluoxetine, fluvoxamine...]), TCA (tricyclic antidepressant [as imipramine, desipramine, amitriptyline...]), MAOI (monoamine oxidase inhibitor [as Clorgyline, Moclobemide, Toloxatone..]), SNRI (serotonin-norepinephrine reuptake inhibitor [as venlafaxine, duloxetine]), NDRI (norepinephrine-dopamine reuptake inhibitor [as bupropion]), SARI (serotonin antagonist and reuptake inhibitor [trazodone]), dopamine reuptake inhibitor [ such as amineptine], lithium.

13. Compound for its use in the treatment and/or the prevention of depressions according to anyone of claims 1 to 12, said compound being liable to be administered during the implementation of a therapy selected from the group consisting of: electroshock therapy, electroconvulsive therapy (ECT), transcranial magnetic stimulation (TMS), repetitive transcranial magnetic stimulation (rTMS), behaviour therapy.

14. Pharmaceutical composition comprising:
(i) at least one compound as defined in anyone of claims 1 to 7, and
(ii) at least one antidepressant drug, in particular selected from the group consisting of: 7,8 DHF (compound with antidepressant-like properties), SSRI (selective serotonin reuptake inhibitor [as citalopram, escitalopram, fluoxetine, fluvoxamine...]), TCA (tricyclic antidepressant [as imipramine, desipramine, amitriptyline...]), MAOI (monoamine oxidase inhibitor [as Clorgyline, Moclobemide, Toloxatone..]), SNRI (serotonin-norepinephrine reuptake inhibitor [as venlafaxine, duloxetine]), NDRI (norepinephrine-dopamine reuptake inhibitor [as bupropion]), SARI (serotonin antagonist and reuptake inhibitor [trazodone]), dopamine reuptake inhibitor [such as amineptine], lithium,
in combination with a pharmaceutically acceptable vehicle.

15. Products comprising:
(i) at least one compound as defined in anyone of claims 1 to 7, in particular the compound of formula V 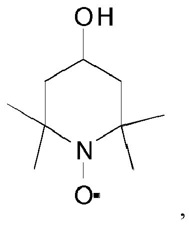 and
(ii) at least one antidepressant drug, in particular selected from the group consisting of: 7,8 DHF (compound with antidepressant-like properties), SSRI (selective serotonin reuptake inhibitor [as citalopram, escitalopram, fluoxetine, fluvoxamine...]), TCA (tricyclic antidepressant [as imipramine, desipramine, amitriptyline...]), MAOI (monoamine oxidase inhibitor [as Clorgyline, Moclobemide, Toloxatone..]), SNRI (serotonin-norepinephrine reuptake inhibitor [as venlafaxine, duloxetine]), NDRI (norepinephrine-dopamine reuptake inhibitor [as bupropion]), SARI (serotonin antagonist and reuptake inhibitor [trazodone]), dopamine reuptake inhibitor [ such as amineptine], lithium,
as a combination for the simultaneous, separate use or successive administration for the treatment and/or the prevention of depressions.
